# FASCICULE DE BREVET EUROPEEN

(11) **EP 1 329 447 B1**
(45) Date de publication et mention de la délivrance du brevet: **27.02.2008**
(21) Numéro de dépôt: 02293040.8
(22) Date de dépôt: 09.12.2002
(51) Int. Cl.: C07C 235/08, A61K 8/42, A61Q 5/12, A61Q 99/00

(54) **Céramides, composition les comprenant et utilisations**
Ceramides, Zusammensetzungen die diese enthalten und ihre Verwendunge
Ceramides, compositions comprising the same and their uses

(30) Priorité: 22.01.2002 FR 0200760
(43) Date de publication de la demande: 23.07.2003
(73) Titulaire: L'ORÉAL, 75008 Paris (FR)
(72) Inventeur: Gaetani, Quintino, 93390 Clichy s/ Bois (FR); Guey, Christèle, 30360 Deaux (FR); Arbey, Eric, 93600 Aulnay sous Bois (FR); Castiel, Isabelle, 78350 Jouy en Josas (FR)
(74) Mandataire: Dodin, Catherine

(56) Documents cités:
- US-A- 5 869 711
- SHIROTA O ET AL: "Phytosphingosines -- A Facile Synthesis and Spectroscopic Protocol for Configurational Assignment" TETRAHEDRON, ELSEVIER SCIENCE PUBLISHERS, AMSTERDAM, NL, vol. 55, no. 48, 26 novembre 1999 (1999-11-26), pages 13643-13658, XP004181506 ISSN: 0040-4020
- DUFFIN, G R ET AL : "Practical syntheses of 13C and 14C-labelled glucosphingolipids" JOURNAL OF THE CHEMICAL SOCIETY, PERKIN TRANSACTIONS 1., 2000, pages 2237-42, XP002215345 CHEMICAL SOCIETY. LETCHWORTH., GB ISSN: 0300-922X

## Description

La présente invention a pour objet de nouveaux céramides, leur procédé de préparation ainsi que leur utilisation, notamment pour le soin de la peau, des cheveux et des ongles en cosmétique ou en dermatologie.

L'épiderme des vertébrés terrestres a évolué de façon à constituer une barrière efficace contre la déperdition en eau, facteur essentiel de survie dans un environnement sec. Il est connu depuis plusieurs décennies que cette fonction barrière est assurée par un ensemble de composés lipidiques tels que les acides gras libres, le cholestérol et ses dérivés ainsi que de nombreux composés céramidiques. Ces derniers sont des molécules extrêmement hydrophobes dont l'agencement physico-chimique dans les espaces intercellulaires dépend étroitement de leurs structures moléculaires de même que les interactions qu'ils peuvent échanger avec les protéines. C'est ainsi que certains céramides sont liés par des liaisons covalentes aux protéines de l'enveloppe des cornéocytes. Ces céramides liés assurent un liant efficace entre les autres lipides et les protéines permettant une meilleure stabilité physico-chimique de l'ensemble. Ce concept de lipides liés (céramides ou acides gras) intervient également dans l'élaboration des annexes de la peau tels que les cheveux et les ongles.

La diversité et la complexité des structures céramidiques sont telles que malgré les recherches entreprises depuis une vingtaine d'années, les connaissances en la matière sont loin d'être satisfaisantes.

US-5,869,711 décrit des composés de type céramides pour les traitements et les soins de la peau, des cheveux, des ongles et des cils en cosmétique ou en dermatologie.

La demanderesse a cherché de nouveaux céramides efficaces dans le renforcement la fonction barrière de l'épiderme. Ce renforcement permet non seulement de mieux protéger la peau contre son dessèchement ou la pénétration de xéno biotiques mais indirectement -par une amélioration des capacités fonctionnelles de la peau- de lutter contre le vieillissement cutané, de limiter ou de ralentir les désordres pigmentaires, de conserver plus longtemps un système immunitaire efficace et d'assurer ainsi une meilleure protection contre les invasions microbiennes ou virales.

La présente demande concerne une composition comprenant, dans un milieu physiologiquement acceptable, au moins un de ces céramides, l'utilisation de ce céramide notamment pour renforcer la barrière lipidique de l'épiderme, et/ou pour améliorer la qualité de l'épiderme. La présente invention a trait à l'utilisation de ces céramides dans des compositions cosmétiques ou pharmaceutiques, notamment dermatologiques.

La demanderesse a ainsi découvert de nouveaux composés qui peuvent être représentés par la formule (I) suivante : 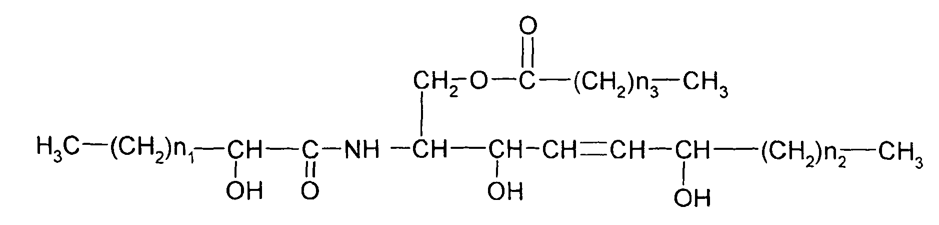 dans laquelle :
n₁ est compris entre 17 et 35, de préférence entre 21 et 30, préférentiellement entre 23 et 28, de préférence encore est égal à 25, 26 ou 27,
n₂ est compris entre 9 et 18, de préférence entre 11 et 15, préférentiellement entre 11 et 13, de préférence encore égal à 11, et
n₃ est compris entre 12 et 18, de préférence entre 14 et 16, de préférence égal à 14.

Ces nouveaux céramides sont constitués d'une base 6-OH-sphingénine amidifiée par un acide gras alpha-hydroxylé à longue chaîne et estérifiée en position 1 par un second acide gras saturé à plus courte chaîne.

Les céramides de la présente invention permettent de renforcer la barrière lipidique de l'épiderme et/ou rétablir ou maintenir l'intégrité du stratum corneum. Ils peuvent donc être employés pour améliorer l'aspect de surface et l'hydratation de la peau, ainsi que pour la protéger, notamment protéger les peaux sèches et rugueuses. Ils peuvent également être utilisés comme agents de nutrition essentiels pour les matières kératiniques (peau, cheveu, cil, ongle).
Par ailleurs, les compositions comprenant ces composés peuvent être avantageusement employées pour améliorer et/ou maintenir, le contenu lipidique de l'épiderme humain, in vivo et in vitro.

Les composés faisant l'objet de l'invention peuvent être isolés à partir de prélèvements lipidiques réalisés par des méthodes non invasives sur des volontaires sains. Ces prélèvements lipidiques sont ensuite soumis à un traitement préparatif et analytique permettant de séparer et d'identifier les familles céramidiques.

Une autre source potentielle des composés de l'invention réside dans l'utilisation d'enzymes telles que des trans-acylases agissant sur des précurseurs ou des modulateurs de ces enzymes.

Les précurseurs sont notamment des composés de formule (II) : 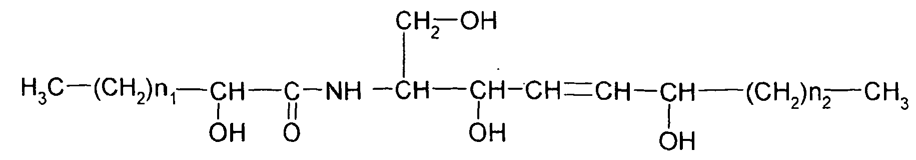 dans laquelle n₁ est compris entre 17 et 35, de préférence entre 21 et 30, préférentiellement entre 23 et 28, de préférence égal à 25, 26 ou 27, n₂ est compris entre 9 et 18, de préférence entre 11 et 15, préférentiellement entre 11 et 13, de préférence encore égal à 11.
Ces précurseurs correspondent aux composés de l'invention non estérifiés en position 1.
Tous ces précurseurs possédant un OH libre pourront également être utilisés pour être accrochés sur des protéines de l'enveloppe via une fonction ester renforçant ainsi la couche d'apprêt lipidique des cornéocytes de la peau ou de ses annexes.

Un objet de la présente invention est une composition comprenant, dans un milieu physiologiquement acceptable, au moins un céramide selon l'invention.
Cette composition est avantageusement une composition cosmétique ou pharmaceutique, notamment dermatologique, comprenant dans un milieu cosmétiquement ou pharmaceutiquement acceptable, au moins un composé de formule (I) selon l'invention.

La composition selon l'invention comprend au moins un composé de formule (I), seul ou en mélange, en une quantité qui peut être comprise entre 0,005 et 20% en poids, de préférence de 0,01 à 10% en poids, notamment 0,05 à 5%, voire 0,1 à 1% en poids, par rapport au poids total de la composition.

Le milieu physiologiquement acceptable dans lequel les composés selon l'invention peuvent être employés, ainsi que ses constituants, leur quantité, la forme galénique de la composition et son mode de préparation, peuvent être choisis par l'homme du métier sur la base de ses connaissances générales en fonction du type de composition recherchée.

La composition peut ainsi comprendre une phase aqueuse qui peut comprendre de l'eau, une eau florale telle que l'eau de bleuet, une eau minérale telle que l'eau de VITTEL, l'eau de LUCAS ou l'eau de LA ROCHE POSAY et/ou une eau thermale.
Ladite phase aqueuse peut comprendre en outre des alcools tels que des monoalcools en C₁-C₆, parmi lesquels on peut citer l'éthanol, le propanol, le butanol, l'isopropanol, l'isobutanol; et/ou des polyols tels que le glycérol, le butylèneglycol, l'isoprène glycol, le propylèneglycol, le polyéthylèneglycol.

La composition selon l'invention peut également comprendre une phase grasse, notamment constituée de corps gras liquides à 25°C, tels que des huiles d'origine animale, végétale, minérale ou synthétique, volatiles ou non; de corps gras solides à 25°C tels que des cires d'origine animale, végétale, minérale ou synthétique; de corps gras pâteux; de gommes; de leurs mélanges.
Les huiles volatiles sont généralement des huiles ayant, à 25°C, une tension de vapeur saturante au moins égale à 0,5 millibar (soit 50 Pa).
Parmi les constituants de la phase grasse, on peut citer :
- les silicones volatiles cycliques ayant de 3 à 8 atomes de silicium et de préférence de 4 à 6. Il s'agit par exemple de la cyclotétradiméthylsiloxane, de la cyclopentadiméthylsiloxane ou de la cyclohexadiméthylsiloxane,
- les cyclocopolymères du type diméthylsiloxane/méthylalkylsiloxane,
- les silicones volatiles linéaires ayant de 2 à 9 atomes de silicium. Il s'agit par exemple de l'hexaméthyldisiloxane ou un PDMS de faible viscosité (1 cSt). On peut encore citer l'hexaméthyldisiloxane et les alkyltrisiloxanes tels que l'hexylheptaméthyltrisiloxane ou l'octylheptaméthyltrisiloxane.
- les huiles volatiles hydrocarbonées, telles que les isoparaffines et notamment l'isododécane;
- les polyalkyl(C₁-C₂₀) siloxanes et notamment ceux à groupements terminaux triméthylsilyle, parmi lesquels on peut citer les polydiméthylsiloxanes linéaires et les alkylméthylpolysiloxanes tels que la cétyldiméthicone (nom CTFA),
- les silicones modifiées par des groupements aliphatiques et/ou aromatiques, éventuellement fluorés, ou par des groupements fonctionnels tels que des groupements hydroxyles, thiols et/ou amines.
- les huiles de silicone phénylées,
- les huiles d'origine animale, végétale ou minérale, et notamment les huiles animales ou végétales formées par des esters d'acide gras et de polyols, en particulier les triglycérides liquides, par exemple les huiles de tournesol, de maïs, de soja, de courge, de pépins de raisin, de sésame, de noisette, d'abricot, d'amandes ou d'avocat; les huiles de poisson, le tricaprocaprylate de glycérol, ou les huiles végétales ou animales de formule R1COOR₂ dans laquelle R₁ représente le reste d'un acide gras supérieur comportant de 7 à 19 atomes de carbone et R₂ représente une chaîne hydrocarbonée ramifiée contenant de 3 à 20 atomes de carbone, par exemple, l'huile de Purcellin; l'huile de paraffine, de vaseline, le perhydrosqualène, l'huile de germes de blé, de calophyllum, de sésame, de macadamia, de pépins de raisin, de colza, de coprah, d'arachide, de palme, de ricin, de jojoba, d'olive ou de germes de céréales; des esters d'acides gras; des alcools; des acétylglycérides; des octanoates, décanoates ou ricinoléates d'alcools ou de polyalcools; des triglycérides d'acides gras; des glycérides;
- les huiles fluorées et perfluorées.
- les gommes de silicones;
- les cires d'origine animale, végétale, minérale ou synthétique telles que les cires microcristallines, la paraffine, le pétrolatum, la vaseline, l'ozokérite, la cire de montan; la cire d'abeilles, la lanoline et ses dérivés; les cires de Candellila, d'Ouricury, de Carnauba, du Japon, le beurre de cacao, les cires de fibres de lièges ou de canne à sucre; les huiles hydrogénées concrètes à 25°C, les ozokérites, les esters gras et les glycérides concrets à 25°C; les cires de polyéthylène et les cires obtenues par synthèse de Fischer-Tropsch; des huiles hydrogénées concrètes à 25°C; des lanolines; des esters gras concrets à 25°C; les cires de silicone; les cires fluorées.

Lorsque la composition selon l'invention se présente sous la forme d'une émulsion, elle peut éventuellement comprendre en outre un tensioactif, de préférence en une quantité de 0,01 à 30% en poids par rapport au poids total de la composition. La composition selon l'invention peut également comprendre au moins un co-émulsionnant qui peut être choisi parmi le monostéarate de sorbitan oxyéthyléné, des alcools gras tels que l'alcool stéarylique ou l'alcool cétylique, ou des esters d'acides gras et de polyols tels que le stéarate de glycéryle.

La composition selon l'invention peut comprendre en outre une phase particulaire qui peut comprendre des pigments et/ou des nacres et/ou des charges habituellement utilisés dans les compositions cosmétiques.
Les pigments peuvent être présents à raison de 0-20 % en poids, par rapport au poids total de la composition, et de préférence à raison de 2-15 %. Ils peuvent être blancs ou colorés, minéraux et/ou organiques, de taille usuelle ou nanométrique. On peut citer, parmi les pigments et les nanopigments minéraux, les dioxydes de titane, de zirconium ou de cérium, ainsi que les oxydes de zinc, de fer ou de chrome, les nanotitanes, le bleu ferrique. Parmi les pigments organiques, on peut citer le noir de carbone, et les laques couramment employées pour conférer aux lèvres et à la peau un effet de maquillage, qui sont des sels de calcium, de baryum, d'aluminium ou de zirconium, de colorants acides tels que les colorants halogéno-acides, azoïques ou anthraquinoniques.
Les nacres peuvent être présentes dans la composition à raison de 0-20% en poids, de préférence à un taux élevé de l'ordre de 2-15% en poids. Parmi les nacres envisageables, on peut citer la nacre naturelle, le mica recouvert d'oxyde de titane, d'oxyde de fer, de pigment naturel ou d'oxychlorure de bismuth ainsi que le mica titane coloré.
Les charges, qui peuvent être présentes dans la composition à raison de 0-20 % en poids, par rapport au poids total de la composition, de préférence 2-10%, peuvent être minérales ou de synthèse, lamellaires ou non lamellaires. On peut citer le talc, le mica, la silice, le kaolin, les poudres de Nylon et de polyéthylène, le Téflon, l'amidon, le nitrure de bore, les microsphères telles que l'Expancel (Nobel Industrie), le polytrap (Dow Corning) et les microbilles de résine de silicone (Tospearls de Toshiba, par exemple).
La composition peut également comprendre des colorants notamment hydrosolubles choisis parmi les colorants usuels du domaine considéré tels que le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle.

La composition selon l'invention peut également comprendre au moins un autre produit actif qui peut être choisi parmi les hydratants ou les humectants comme la glycérine, le sorbitol, le pentaérythritol, l'acide pyrrolidone carboxylique et ses sels; les adoucissants; les produits pour le traitement d'affections cutanées; les filtres solaires; les germicides; les conservateurs; les antioxydants; les agents de brunissage artificiel tels que la dihydroxyacétone, l'érythrulose, le glycéraldéhyde, les gamma-dialdéhydes tels que l'aldéhyde tartrique, ces composés étant éventuellement associés à des colorants; les filtres solaires notamment hydrosolubles ou liposolubles; les antiperspirants, les déodorants, les astringents; les produits rafraîchissants, toniques, cicatrisants, kératolytiques, dépilatoires; les eaux parfumées; les extraits de tissus végétaux, tels que les polysaccharides; les agents antipelliculaires; les agents antiséborrhéiques; les oxydants tels que des agents de décoloration comme l'eau oxygénée; les réducteurs tels que l'acide thioglycolique et ses sels; les substances destinées à améliorer l'état des peaux sèches ou séniles, les tocophérols, les vitamines E, F ou A et leurs esters, l'acide rétinoïque, les antioxydants, les acides gras essentiels, l'acide glycyrrhétinique, les kératolytiques et les caroténoïdes.

La composition de l'invention peut comprendre par ailleurs des actifs pouvant aider à prévenir ou à améliorer les matières kératiniques, notamment la peau, et encore mieux les peaux sèches, rugueuses et/ou abîmées, tels que la sphingénine, la sphinganine ou la 4-OH-sphinganine; les céramides; les glycocéramides; les acides gras en C14-30; les triglycérides; les stérols comme le cholestérol.

De façon connue, la composition selon l'invention peut comprendre les adjuvants habituels dans le domaine considéré, tels que les gélifiants hydrophiles ou lipophiles, les additifs hydrophiles ou lipophiles, les actifs notamment cosmétiques ou pharmaceutiques hydrophiles ou lipophiles, les solvants, les parfums, les propulseurs, les absorbeurs d'odeur. La nature et la quantité de ces adjuvants peuvent être choisies par l'homme du métier, sur la base de ses connaissances générales, de manière à obtenir la forme de présentation désirée pour la composition. En tout état de cause, l'homme du métier veillera à choisir tous les éventuels composés complémentaires et/ou leur quantité, de manière telle que les propriétés avantageuses de la composition selon l'invention ne soient pas, ou substantiellement pas, altérées par l'adjonction envisagée.

Les compositions selon l'invention peuvent ainsi se présenter sous forme d'émulsions, de lotions hydroalcooliques, huileuses ou oléoalcooliques, de gels, de dispersions ou de bâtonnets solides, de sprays ou de mousses aérosol. Pour une application sur la peau, la composition peut avoir la forme notamment de solution aqueuse ou huileuse; de dispersion du type lotion ou sérum; d'émulsions de consistance liquide ou semi-liquide du type lait obtenues par dispersion d'une phase grasse dans une phase aqueuse (H/E) ou inversement (E/H); de suspensions ou émulsions de consistance molle du type crème ou gel aqueux ou anhydres; de microcapsules ou microparticules; de dispersions vésiculaires de type ionique et/ou non ionique.
Pour une application sur les cheveux, la composition peut être sous forme de solutions aqueuses, alcooliques ou hydroalcooliques; sous forme de crèmes, de gels, d'émulsions, de mousses; sous forme de compositions pour aérosol comprenant également un agent propulseur sous pression.

Les compositions sont par exemple des lotions, des laits ou des crèmes émollients, des laits ou des crèmes pour les soins de la peau ou des cheveux, des crèmes, des lotions ou des laits démaquillants, des bases de fond de teint, des lotions, des laits ou des crèmes antisolaires, des lotions, des laits ou des crèmes de bronzage artificiel, des crèmes ou des mousses de rasage, des lotions après rasage, des shampooings ou des mascaras.
Ces compositions peuvent également se présenter sous la forme de bâtons pour les lèvres destinés soit à les colorer, soit à éviter les gerçures, ou de produits de maquillage pour les yeux ou de fards et fonds de teint pour le visage.

Les compositions selon l'invention peuvent notamment se présenter sous la forme :
- d'un produit de soin, de traitement ou de protection, des matières kératiniques, et notamment de la peau du visage ou du corps y compris le cuir chevelu, tel qu'une composition de soin (de jour, de nuit, hydratante) du visage ou du corps; une composition anti-rides ou anti-age pour le visage; une composition matifiante pour le visage; une composition pour les peaux irritées; une composition démaquillante; un lait pour le corps, notamment hydratant éventuellement après-soleil;
- d'une composition de protection solaire, de bronzage artificiel (autobronzant) ou de soin après-soleil;
- d'une composition capillaire, et notamment une crème ou un gel protecteur solaire; une composition de soin du cuir chevelu, notamment anti-chute ou repousse des cheveux; un shampooing antiparasitaire;
- d'un produit de maquillage des matières kératiniques tel qu'un fond de teint, une crème teintée, un fard à joues ou à paupières, une poudre libre ou compacte, un stick anti-cernes, un stick camouflant, un rouge à lèvres, un soin des lèvres; un vernis à ongles, un soin des ongles, un mascara, un mascara traitant, un eye-liner.

Les compositions selon l'invention trouvent une application préférée comme 10. composition pour le soin des matières kératiniques, notamment de la peau, des ongles, des cils, des cheveux, et en particulier de la peau sèche et/ou rugueuse et/ou abîmée, et particulièrement comme composition de soin de la peau du visage, de type hydratante, et comme composition de protection solaire ou après-soleil.

La présente invention a également pour objet l'utilisation des composés de formule (I) pour le renforcement de la fonction barrière de la peau et pour le renforcement des ongles et des cheveux.
Plus précisément, l'invention a pour objet l'utilisation d'au moins un composé de formule (I) ou d'une composition le comprenant, pour la préparation d'une composition, notamment cosmétique ou pharmaceutique, destinée à renforcer la barrière lipidique de l'épiderme, notamment de la peau sèche et/ou rugueuse et/ou abîmée, et/ou à rétablir ou maintenir l'intégrité du stratum corneum et/ou à améliorer l'aspect de surface et/ou l'hydratation de la peau, et/ou à protéger la peau, notamment les peaux sèches et rugueuses, et/ou comme agent de nutrition essentiel pour les matières kératiniques (peau, cheveu, cil, ongle) et/ou à améliorer et/ou maintenir le contenu lipidique de l'épiderme humain, in vivo et in vitro, et/ou au traitement des affections dermatologiques se traduisant par un déséquilibre de la fonction barrière ou de l'homéostasie épidermique.

La présente invention vise également l'utilisation d'au moins un composé de formule (I) ou d'une composition le comprenant, pour le traitement des affections dermatologiques se traduisant par un déséquilibre de la fonction barrière ou de l'homéostasie épidermique.

Enfin, un autre objet de l'invention est constitué par un procédé de traitement cosmétique de la peau, des cheveux, des cils ou des ongles consistant à appliquer sur ces derniers une quantité suffisante d'une composition comprenant au moins un céramide selon l'invention.

L'invention est illustrée plus en détails par les exemples suivants.

### Exemple 1

### Prélèvement lipidique

Les prélèvements ont lieu sur une cohorte de 22 femmes (volontaires sains) d'âge moyen 33,8 ans +/- 8,8 et présentant une peau qualifiée de normale par les cliniciens. Les prélèvements sont réalisés sur l'avant bras à l'aide d'une turbine après nettoyage de la peau au moyen d'un coton imbibé d'éther afin d'éliminer les traces de sébum. La chambre d'extraction de la turbine, remplie avec 10 ml de mélange hexane/éthanol (2/3) est appliquée sur une surface de 12,56 cm². Le mélange est agité durant 1 minute puis est collecté à l'aide d'une seringue en verre puis stocké dans un récipient en verre à -20 °C.
Trois prélèvements sont ainsi réalisés sur l'avant bras de chaque personne. Les échantillons sont ensuite rassemblés, évaporés à sec à l'aide d'un évaporateur rotatif, repris dans 1 ml de chloroforme /méthanol (2/1) et conservé à -20 °C. L'extrait sec correspondant à l'ensemble de ces prélèvements est de 36,4 mg et représente une surface extraite de 829 cm².

### Séparation et isolement des céramides

Dans un premier temps, on sépare les céramides des autres catégories de lipides en déposant l'échantillon lipidique précédemment obtenu sur une cartouche de silice phase normale (gel de silice 60). Après élimination des lipides neutres par 10 ml de chloroforme contenant 1% d'acide acétique, les céramides sont élués avec 10 ml de mélange chloroforme /méthanol (95/5). Les céramides sont ensuite stockés dans 1 ml de chloroforme à -20 °C.

Dans un second temps, les différentes céramides sont séparées par chromatographie couche mince dans les conditions suivantes :
On dépose 1,7 mg du mélange de céramides obtenues à l'issue de la première étape sur une plaque de silice Whatman LK5 de 20x20 cm et on procède à 2 élutions successives avec un mélange chloroforme /méthanol /acide acétique dans les rapports 190/5/1 pour la première élution et 190/9/1 pour la seconde élution.

Les classes de céramides sont repérées en ultraviolet (à 254 nm) après vaporisation sur la plaque d'une solution de primuline à 5 mg/100 ml. Cette observation permet de délimiter 10 zones contiguës sur la plaque de silice numérotées de 1 à 10 en allant de la zone la plus éluée à la zone la moins éluée. La silice de chacune de ces zones est grattée, récupérée et extraite à plusieurs reprises avec un mélange de chloroforme /méthanol (2/1). Les phases organiques sont rassemblées et lavées à l'eau puis évaporées à sec pour obtenir les composés céramidiques purs. Une partie de ces céramides est ensuite remise en solution dans un mélange chloroforme/méthanol (2/1) pour identification analytique.

On récupère dans la tache no1 de la plaque de silice, des céramides de l'invention telles que n₁ est compris entre 17 et 35, n₂ est compris entre 9 et 18, et n₃ est compris entre 12 et 18.

### Analyse structurale des composés céramidiques

L'échantillon destiné à l'analyse est réparti en deux fractions.

On réalise une dérivation de la première fraction céramides au moyen du chlorure de benzoyle. Les dérivés benzoylés ainsi obtenus sont séparés en chromatographie liquide haute performance et injectés dans un spectrographe de masse en sortie de colonne par couplage HPLC-MS.

La deuxième fraction subit une hydrolyse alcaline pour libérer les bases sphingoïdes contenues dans les céramides. Les bases libérées sont dérivées à l'orthophtalaldéhyde avant d'être séparées en HPLC avec détection en fluorescence.
L'ensemble des résultats analytiques ainsi obtenu permet d'attribuer une structure moléculaire précise à chaque céramide présent dans l'échantillon.

Dans le mélange, on isole une fraction A contenant les céramides telles que n₁ est compris entre 25 et 27, n₂ est égal à 11, et n₃ est égal à 14.

### Exemple 2

On prépare une émulsion huile-dans-eau, destinée à être une crème de soin pour la peau, comprenant (% en poids) :
- Stéarate de glycérol 2 %
- Monostéarate de sorbitan 1 %
- gélifiant (Carbopol 940 ® vendu par Goodrich) 0,4 %
- fraction liquide de graisse de karité 24 %
- Fraction A de céramides de l'exemple 1 0,2 %
- Alcool cétylique 0,5 %
- Alcool stéarique 1,4 %
- triéthanolamine 0,7 %
- antioxydants qs
- parfum qs
- Conservateurs qs
- Eau qsp 100%

### Exemple 3

On prépare un rouge à lèvres comprenant (% en poids) :
- Huile de ricin 25 %
- lanoline 20 %
- cires 15 %
- Fraction A de céramides de l'exemple 1 20 %
- pigments 8 %
- huile de vaseline qsp 100%

### Exemple 4

On prépare un soin pour les cheveux comprenant :
- décaméthylcyclopentasiloxane 25 g
- Fraction A de céramides de l'exemple 1 0,1 g
- Mélange d'un polydiméthylsiloxane hydroxylé en bout de chaîne et de cyclométhicone (Q2-1401 de DOW CORNING) 65 g
- benzoate d'alcools gras en C12-C15 (FINSOLV TN) 4,95 g

## Revendications

1. Composé de formule (I) suivante : 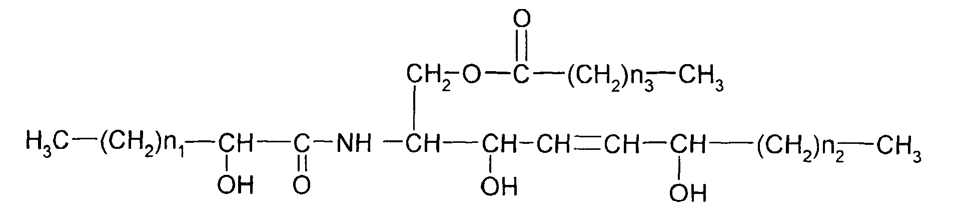 dans laquelle n₁ est compris entre 17 et 35, n₂ est compris entre 9 et 18, et n₃ est compris entre 12 et 18.

2. Composé selon la revendication 1, **caractérisé par le fait que** n₁ est compris entre 21 et 30, préférentiellement entre 23 et 28, de préférence encore est égal à 25, 26 ou 27.

3. Composé selon l'une des revendications précédentes, **caractérisé par le fait que** n₂ est compris entre 11 et 15, préférentiellement entre 11 et 13, de préférence encore égal à 11.

4. Composé selon l'une des revendications précédentes, **caractérisé par le fait que** n₃ est compris entre 14 et 16, de préférence égal à 14.

5. Composition comprenant, dans un milieu physiologiquement acceptable, au moins un composé selon l'une des revendications 1 à 4.

6. Composition selon la revendication 5, se présentant sous la forme d'une composition cosmétique ou pharmaceutique, comprenant un milieu cosmétiquement ou pharmaceutiquement acceptable.

7. Composition selon l'une des revendications 5 à 6, dans laquelle le composé de formule (1) est présent en une quantité de 0,005 à 20% en poids, de préférence de 0,01 à 10% en poids, notamment 0,05 à 5%, voire 0,1 à 1 % en poids, par rapport au poids total de la composition.

8. Composition selon l'une des revendications 5 à 7, comprenant au moins un constituant choisi parmi l'eau, les monoalcools en C₁-C₆, les polyols, les huiles d'origine animale, végétale, minérale ou synthétique, volatiles ou non; les cires d'origine animale, végétale, minérale ou synthétique; les corps gras pâteux; les gommes; les tensioactifs, les co-émulsionnants, les pigments, les nacres, les charges, les colorants, les gélifiants hydrophiles ou lipophiles, les additifs hydrophiles ou lipophiles, les actifs notamment cosmétiques ou pharmaceutiques hydrophiles ou lipophiles, les solvants, les parfums, les propulseurs, les absorbeurs d'odeur; les hydratants ou les humectants; les adoucissants; les produits pour le traitement d'affections cutanées; les filtres solaires; les germicides; les conservateurs; les antioxydants; les agents de brunissage artificiel; les filtres solaires notamment hydrosolubles ou liposolubles; les antiperspirants, les déodorants, les astringents; les produits rafraîchissants, toniques, cicatrisants, kératolytiques, dépilatoires; les eaux parfumées; les extraits de tissus végétaux; les agents antipelliculaires; les agents antiséborrhéiques; les oxydants tels que des agents de décoloration; les réducteurs; les substances destinées à améliorer l'état des peaux sèches ou séniles, les tocophérols, les vitamines E, F ou A et leurs esters, l'acide rétinoïque, les acides gras essentiels, l'acide glycyrrhétinique, les kératolytiques et les caroténoïdes; des actifs pouvant aider à prévenir ou à améliorer les matières kératiniques, tels que la sphingénine, la sphinganine ou la 4-OH-sphinganine; les céramides; les glycocéramides; les acides gras en C14-30; les triglycérides; les stérols comme le cholestérol.

9. Composition selon l'une des revendications 5 à 8, se présentant sous la forme
- d'un produit de soin, de traitement ou de protection, des matières kératiniques, et notamment de la peau du visage ou du corps y compris le cuir chevelu, tel qu'une composition de soin (de jour, de nuit, hydratante) du visage ou du corps; une composition anti-rides ou anti-age pour le visage; une composition matifiante pour le visage; une composition pour les peaux irritées; une composition démaquillante; un lait pour le corps, notamment hydratant éventuellement après-soleil;
- d'une composition de protection solaire, de bronzage artificiel (autobronzant) ou de soin après-soleil;
- d'une composition capillaire, et notamment une crème ou un gel protecteur solaire; une composition de soin du cuir chevelu, notamment anti-chute ou repousse des cheveux; un shampooing antiparasitaire;
- d'un produit de maquillage des matières kératiniques tel qu'un fond de teint, une crème teintée, un fard à joues ou à paupières, une poudre libre ou compacte, un stick anti-cernes, un stick camouflant, un rouge à lèvres, un soin des lèvres; un vernis à ongles, un soin des ongles, un mascara, un mascara traitant, un eye-liner.

10. Composition selon l'une des revendications 5 à 9, se présentant sous la forme d'une composition pour le soin des matières kératiniques, notamment de la peau, des ongles, des cils, des cheveux, et en particulier de la peau sèche et/ou rugueuse et/ou abîmée.

11. Utilisation cosmétique d'au moins un composé de formule (I) tel que défini dans l'une des revendications 1 à 4,
- pour renforcer la fonction barrière de la peau, notamment de la peau sèche et/ou rugueuse et/ou abîmée, et/ou
- pour renforcer les ongles et/ou les cheveux, et/ou
- pour rétablir et/ou maintenir l'intégrité du stratum corneum, et/ou
- pour améliorer l'aspect de surface et/ou l'hydratation de la peau,
- pour protéger la peau, notamment les peaux sèches et rugueuses, et/ou
- comme agent de nutrition essentiel pour les matières kératiniques (peau, cheveu, cil, ongle), et/ou
- pour améliorer et/ou maintenir le contenu lipidique de l'épiderme humain, in vivo et in vitro.

12. Utilisation d'au moins un composé de formule (I) tel que défini dans l'une des revendications 1 à 4, dans une composition cosmétique destinée à
- renforcer la fonction barrière de la peau, notamment de la peau sèche et/ou rugueuse et/ou abîmée, et/ou
- renforcer les ongles et/ou les cheveux, et/ou
- rétablir et/ou maintenir l'intégrité du stratum corneum, et/ou
- améliorer l'aspect de surface et/ou l'hydratation de la peau,
- protéger la peau, notamment les peaux sèches et rugueuses, et/ou
- améliorer et/ou maintenir le contenu lipidique de l'épiderme humain, in vivo et in vitro.

13. Utilisation d'au moins un composé de formule (I) tel que défini dans l'une des revendications 1 à 4, pour la préparation d'une composition pharmaceutique destinée à
- renforcer la fonction barrière de la peau, notamment de la peau sèche et/ou rugueuse et/ou abîmée, et/ou
- renforcer les ongles et/ou les cheveux, et/ou
- rétablir et/ou maintenir l'intégrité du stratum corneum, et/ou
- améliorer l'aspect de surface et/ou l'hydratation de la peau,
- protéger la peau, notamment les peaux sèches et rugueuses, et/ou
- améliorer et/ou maintenir le contenu lipidique de l'épiderme humain, in vivo et in vitro, et/ou
- traiter les affections dermatologiques se traduisant par un déséquilibre de la fonction barrière ou de l'homéostasie épidermique.

14. Procédé de traitement cosmétique de la peau, des cheveux, des cils ou des ongles, **caractérisé par le fait qu'**il consiste à appliquer sur la peau, les cheveux, les ongles ou les cils une quantité suffisante d'une composition cosmétique selon l'une quelconque des revendications 6 à 10.

## Claims

1. Compound of formula (I) below: 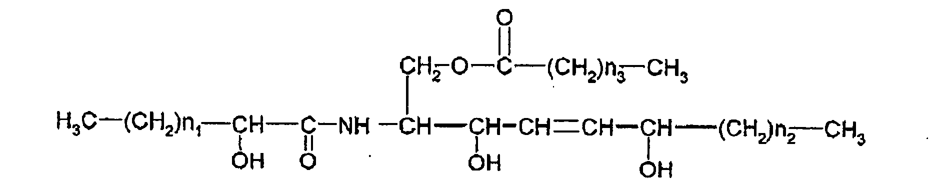 in which n₁ is between 17 and 35, n₂ is between 9 and 18 and n₃ is between 12 and 18.

2. Compound according to Claim 1, **characterized in that** n₁ is between 21 and 30, preferentially between 23 and 28, more preferably is equal to 25, 26 or 27.

3. Compound according to either of the preceding claims, **characterized in that** n₂ is between 11 and 15, preferentially between 11 and 13, more preferably equal to 11.

4. Compound according to one of the preceding claims, **characterized in that** n₃ is between 14 and 16, preferably equal to 14.

5. Composition comprising, in a physiologically acceptable medium, at least one compound according to one of Claims 1 to 4.

6. Composition according to Claim 5, which is provided in the form of a cosmetic or pharmaceutical composition, comprising a cosmetically or pharmaceutically acceptable medium.

7. Composition according to either of Claims 5 and 6, in which the compound of formula (I) is present in an amount of 0.005 to 20% by weight, preferably of 0.01 to 10% by weight, in particular 0.05 to 5%, indeed even 0.1 to 1% by weight, with respect to the total weight of the composition.

8. Composition according to one of Claims 5 to 7, comprising at least one constituent chosen from water, C₁-C₆ monoalcohols, polyols, volatile or non-volatile oils of animal, vegetable, mineral or synthetic origin; waxes of animal, vegetable, mineral or synthetic origin; pasty fatty substances; gums; surfactants, coemulsifiers, pigments, pearlescent agents, fillers, dyes, hydrophilic or lipophilic gelling agents, hydrophilic or lipophilic additives, active principles, in particular hydrophilic or lipophilic cosmetic or pharmaceutical active principles, solvents, fragrances, propellants, odour absorbers; moisturizers or humectants; softeners; products for the treatment of skin conditions; sunscreens; germicides; preservatives; antioxidants; artificial browning agents; sunscreens, in particular water-soluble or fat-soluble sunscreens; antiperspirants, deodorants or astringents; cooling, toning, healing, keratolytic or depilatory products; scented waters; extracts from plant tissues; antidandruff agents; antiseborrhoeic agents; oxidizing agents, such as bleaching agents; reducing agents; substances intended to improve the condition of dry or senile skin, tocopherols, vitamins E, F or A and their esters, retinoic acid, essential fatty acids, glycyrrhetinic acid, keratolytic agents and carotenoids; active principles which can help in preventing or in improving keratinous substances, such as sphingenine, sphinganine or 4-OH-sphinganine; ceramides; glycoceramides; C₁₄₋₃₀ fatty acids; triglycerides; or sterols, such as cholesterol.

9. Composition according to one of Claims 5 to 8, which is provided in the form
- of a product for caring for, treating or protecting keratinous substances and in particular the skin of the face or body, including the scalp, such as a care (day, night or moisturizing) composition for the face or body; an anti-wrinkle or anti-age composition for the face; a mattifying composition for the face; a composition for irritated skin; a make-up-removing composition; or an optionally aftersun, in particular moisturizing, body milk;
- of a sun protection, artificial tanning (self-tanning) or aftersun care composition;
- of a hair composition and in particular a cream or a gel for protecting from the sun; a composition for caring for the scalp, in particular a composition for combating hair loss or for promoting hair regrowth; or an antiparasitic shampoo;
- of a product for making up keratinous substances, such as a foundation, a tinted cream, a blusher, an eyeshadow, a loose or compact powder, a concealer stick, a cover stick, a lipstick or a lipcare product; a nail varnish, a nail care product, a mascara, a treating mascara or an eyeliner.

10. Composition according to one of Claims 5 to 9, which is provided in the form of a composition for caring for keratinous substances, in particular the skin, nails, eyelashes or hair, and especially dry and/or rough and/or damaged skin.

11. Cosmetic use of at least one compound of formula (I) as defined in one of Claims 1 to 4,
- for strengthening the barrier role of the skin, in particular of dry and/or rough and/or damaged skin, and/or
- for strengthening the nails and/or hair, and/or
- for re-establishing and/or maintaining the integrity of the stratum corneum, and/or
- for improving the surface appearance and/or the moisturizing of the skin,
- for protecting the skin, in particular dry and rough skin, and/or
- as essential nutritional agent for keratinous substances (skin, hair, eyelash, nail), and/or
- for improving and/or maintaining the lipid content of the human epidermis, in vivo and in vitro.

12. Use of at least one compound of formula (I) as defined in one of Claims 1 to 4 in a cosmetic composition intended to
- strengthen the barrier role of the skin, in particular of dry and/or rough and/or damaged skin, and/or
- strengthen the nails and/or hair, and/or
- re-establish and/or maintain the integrity of the stratum corneum, and/or
- improve the surface appearance and/or the moisturizing of the skin,
- protect the skin, in particular dry and rough skin, and/or
- improve and/or maintain the lipid content of the human epidermis, in vivo and in vitro.

13. Use of at least one compound of formula (I) as defined in one of Claims 1 to 4 in the preparation of a pharmaceutical composition intended to
- strengthen the barrier role of the skin, in particular dry and/or rough and/or damaged skin, and/or
- strengthen the nails and/or hair, and/or
- re-establish and/or maintain the integrity of the stratum corneum, and/or
- improve the surface appearance and/or the moisturizing of the skin,
- protect the skin, in particular dry and rough skin, and/or
- improve and/or maintain the lipid content of the human epidermis, in vivo and in vitro, and/or
- treat dermatological conditions reflected by an imbalance in the barrier role or in epidermal homeostasis.

14. Process for the cosmetic treatment of the skin, hair, eyelashes or nails, **characterized in that** it consists in applying, to the skin, hair, nails or eyelashes, a sufficient amount of a cosmetic composition according to any one of Claims 6 to 10.

## Patentansprüche

1. Verbindung der folgenden Formel (I): 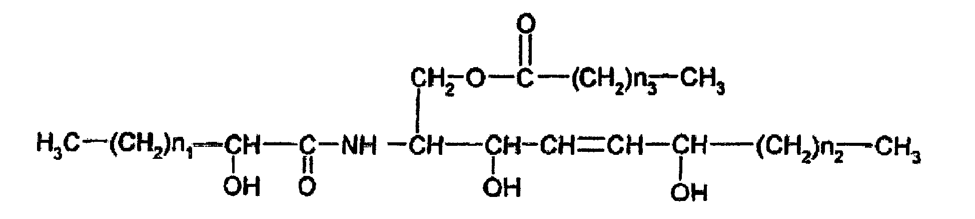 worin n₁ im Bereich von 17 bis 35 liegt, n₂ im Bereich von 9 bis 18 liegt und n₃ im Bereich von 12 bis 18 liegt.

2. Verbindung nach Anspruch 1, **dadurch gekennzeichnet, dass** n₁ im Bereich von 21 bis 30 und vorzugsweise im Bereich von 23 bis 28 liegt und noch bevorzugter 25, 26 oder 27 beträgt.

3. Verbindung nach einem der vorhergehenden Ansprüche, **dadurch gekennzeichnet, dass** n₂ im Bereich von 11 bis 15 und vorzugsweise 11 bis 13 liegt und noch bevorzugter 11 beträgt.

4. Verbindung nach einem der vorhergehenden Ansprüche, **dadurch gekennzeichnet, dass** n₃ im Bereich von 14 bis 16 liegt und vorzugsweise 14 beträgt.

5. Zusammensetzung, die in einem physiologisch akzeptablen Medium mindestens eine Verbindung nach einem der Ansprüche 1 bis 4 enthält.

6. Zusammensetzung nach Anspruch 5, **dadurch gekennzeichnet, dass** sie in Form einer kosmetischen oder pharmazeutischen Zusammensetzung vorliegt, die ein kosmetisch oder pharmazeutisch akzeptables Medium enthält.

7. Zusammensetzung nach einem der Ansprüche 5 bis 6, wobei die Verbindung der Formel (I) in einer Menge von 0,005 bis 20 Gew.-%, vorzugsweise 0,01 bis 10 Gew.-%, insbesondere 0,05 bis 5 % und sogar 0,1 bis 1 Gew.-%, bezogen auf das Gesamtgewicht der Zusammensetzung, enthalten ist.

8. Zusammensetzung nach einem der Ansprüche 5 bis 7, die mindestens einen Bestandteil enthält, der unter Wasser, C₁₋₆-Monoalkoholen, Polyolen, Ölen tierischer, pflanzlicher, mineralischer oder synthetischer Herkunft, die flüchtig oder nichtflüchtig sind; Wachsen tierischer, pflanzlicher, mineralischer oder synthetischer Herkunft; pastösen Fettsubstanzen; Gummis; grenzflächenaktiven Stoffen, Coemulgatoren, Pigmenten, Perlglanzpigmenten, Füllstoffen, Farbmitteln, hydrophilen oder lipophilen Gelbildnern, hydrophilen oder lipophilen Zusatzstoffen, Wirkstoffen und insbesondere kosmetischen oder pharmazeutischen Wirkstoffen, die hydrophil oder lipophil sind, Lösemitteln, Parfums, Treibmitteln, Geruchsabsorbern; Hydratisierungsmitteln oder Feuchthaltemitteln; beruhigenden Stoffen; Produkten zur Behandlung von Hauterkrankungen; Sonnenschutzfiltern; Germiziden; Konservierungsmitteln; Antioxidantien; Stoffen für die künstliche Bräunung; Sonnenschutzfiltern und insbesondere wasserlöslichen oder fettlöslichen Sonnenschutzfiltern; Antiperspirantien, Deodorants, Adstringentien; erfrischenden Produkten, tonisierenden Produkten, Wundheilungsmitteln, Keratolytika, Haarentfernungsmitteln; Parfums; Extrakten von Pflanzengeweben; Antischuppenmitteln; Wirkstoffen gegen Seborrhoe; oxidierenden Stoffen, wie Entfärbungsmitteln; Reduktionsmitteln; Substanzen, die zur Verbesserung des Zustands von trockener Haut oder reifer Haut vorgesehen sind, Tocopherolen, Vitaminen E, F oder A und ihren Estern, Retinsäure, essentiellen Fettsäuren, Glycyrrhetinsäure, Keratolytika und Carotinoiden; Wirkstoffen, die dazu beitragen können, Keratinsubstanzen zu verbessern oder ihrer Beeinträchtigung vorzubeugen, wie Sphingenin, Sphinganin oder 4-OH-Sphinganin; Ceramiden; Glycoceramiden; Fettsäuren mit 14 bis 30 Kohlenstoffatomen; Triglyceriden; Sterolen wie Cholesterol, ausgewählt ist.

9. Zusammensetzung nach einem der Ansprüche 5 bis 8, die vorliegt als
- Produkt zur Pflege, zur Behandlung oder zum Schutz von Keratinsubstanzen und insbesondere der Haut des Gesichts oder des Körpers einschließlich der Kopfhaut, wie beispielsweise in Form einer Zusammensetzung zur Pflege (Tagespflege, Nachtpflege, hydratisierende Pflege) des Gesichts oder des Körpers; Zusammensetzung gegen Falten oder gegen Alterung für das Gesicht; mattierende Zusammensetzung für das Gesicht; Zusammensetzung für gereizte Haut; Zusammensetzung zum Abschminken; Körpermilch, insbesondere als hydratisierende Körpermilch, gegebenenfalls zur Anwendung nach Sonnenbestrahlung;
- Zusammensetzung zum Sonnenschutz, zur künstlichen Bräunung (Selbstbräunungsmittel) oder als pflegende After-Sun-Zusammensetzung;
- Zusammensetzung für die Haarbehandlung und insbesondere als Creme oder Gel zum Sonnenschutz; Zusammensetzung für die Pflege der Kopfhaut, insbesondere gegen Haarausfall oder für das Haarwachstum; antiparasitäres Haarwaschmittel;
- Produkt zum Schminken von Keratinsubstanzen, wie beispielsweise als Make-up, getönte Creme, Wangenrouge, Lidschatten, loser Puder oder kompaktierter Puder, Stift gegen Augenringe, Abdeckstift, Lippenstift, Pflegeprodukt für die Lippen; Nagellack, Nagelpflege, Mascara, Mascara mit Behandlungswirkung, Eyeliner.

10. Zusammensetzung nach einem der Ansprüche 5 bis 9, die in Form einer Zusammensetzung für die Pflege von Keratinsubstanzen und insbesondere für die Pflege der Haut, der Nägel, der Wimpern und der Haare und insbesondere zur Pflege von trockener Haut und/oder geröteter Haut und/oder geschädigter Haut vorliegt.

11. Kosmetische Verwendung mindestens einer Verbindung der Formel (I) nach einem der Ansprüche 1 bis 4,
- um die Barrierefunktion der Haut zu stärken, insbesondere bei trockener Haut und/oder geröteter Haut und/oder geschädigter Haut, und/oder
- um die Nägel und/oder die Haare zu stärken, und/oder
- um die Integrität des *Stratum corneum* wiederherzustellen, und/oder aufrecht zu erhalten, und/oder
- um das Aussehen der Hautoberfläche und/oder die Hydratisierung der Haut zu verbessern,
- um die Haut und insbesondere trockene Haut und gerötete Haut zu schützen, und/oder
- als wesentlicher Nährstoff für die Keratinsubstanzen (Haut, Haare, Wimpern, Nagel), und/oder
- um den Lipidgehalt der menschlichen Epidermis *in vivo* und *in vitro* zu verbessern und/oder aufrecht zu erhalten.

12. Verwendung mindestens einer Verbindung der Formel (I) nach einem der Ansprüche 1 bis 4 in einer kosmetischen Zusammensetzung, die dazu vorgesehen ist,
- die Barrierefunktion der Haut und insbesondere von trockener Haut und/oder geröteter Haut und/oder geschädigter Haut zu verstärken, und/oder
- die Nägel und/oder die Haare zu stärken, und/oder
- die Integrität des *Stratum corneum* wiederherzustellen, und/oder aufrecht zu erhalten, und/oder
- das Aussehen der Hautoberfläche und/oder die Hydratisierung der Haut zu verbessern,
- die Haut und insbesondere trockene Haut und gerötete Haut zu schützen, und/oder
- den Lipidgehalt der menschlichen Epidermis *in vivo* und *in vitro* zu verbessern und/oder aufrecht zu erhalten.

13. Verwendung mindestens einer Verbindung der Formel (I) nach einem der Ansprüche 1 bis 4 für die Herstellung einer pharmazeutischen Zusammensetzung, die vorgesehen ist,
- die Barrierefunktion der Haut und insbesondere von trockener Haut und/oder geröteter Haut und/oder geschädigter Haut zu verstärken, und/oder
- die Nägel und/oder die Haare zu stärken, und/oder
- die Integrität des *Stratum corneum* wiederherzustellen, und/oder aufrecht zu erhalten, und/oder
- das Aussehen der Hautoberfläche und/oder die Hydratisierung der Haut zu verbessern,
- die Haut und insbesondere trockene Haut und gerötete Haut zu schützen, und/oder
- den Lipidgehalt der menschlichen Epidermis *in vivo* und *in vitro* zu verbessern und/oder aufrecht zu erhalten, und/oder
- dermatologische Erkrankungen zu behandeln, die sich durch ein Ungleichgewicht der Barrierefunktion oder der Homöostase der Epidermis ausdrücken.

14. Verfahren zur kosmetischen Behandlung der Haut, der Haare, der Wimpern oder der Nägel, **dadurch gekennzeichnet, dass** es darin besteht, auf die Haut, die Haare, die Nägel oder die Wimpern eine wirksame Menge einer kosmetischen Zusammensetzung nach einem der Ansprüche 6 bis 10 aufzutragen.
